# EUROPÄISCHE PATENTSCHRIFT

(11) **EP 0 636 016 B1**
(45) Veröffentlichungstag und Bekanntmachung des Hinweises auf die Patenterteilung: **11.06.1997**
(21) Anmeldenummer: 94906121.2
(22) Anmeldetag: 18.02.1994
(51) Int. Cl.: A61F 2/36

(54) **SCHAFTKOMPONENTE FÜR EINE ENDOGELENKPROTHESE**
SHAFT COMPONENT FOR A JOINT ENDOPROSTHESIS
TIGE D'ENDOPROTHESE D'ARTICULATION

(30) Priorität: 18.02.1993 CH 506/93
(43) Veröffentlichungstag der Anmeldung: 01.02.1995
(73) Patentinhaber: SCHAWALDER, Peter, CH-3033 Wohlen b. Berm (CH)
(72) Erfinder: SCHAWALDER, Peter, CH-3033 Wohlen b. Berm (CH)
(74) Vertreter: Lusuardi, Werther Giovanni, Dr.
(86) Internationale Anmeldenummer: CH9400037
(87) Internationale Veröffentlichungsnummer: WO9418911

(56) Entgegenhaltungen:
- EP-A- 0 228 511
- EP-A- 0 295 200
- EP-A- 0 393 608
- FR-A- 2 634 371
- US-A- 4 051 559
- US-A- 4 384 373
- US-A- 5 002 578

## Beschreibung

Die Erfindung bezieht sich auf eine Schaftkomponente für eine Endogelenkprothese zur Implantation in einen Röhrenknochen gemäss der Gattung des Patentanspruchs 1.

Aus der US-A-4 051 559 ist bereits eine gattungsgemässe Schaftkomponente für eine Hüfttotalprothese bekannt. Sie besteht im wesentlichen aus einem im Knochen zu fixierenden Verankerungsteil und einem separaten am Verankerungsteil lösbar befestigbaren Gelenkteil. Nachteilig bei dieser bekannten Schaftkomponente ist die äussere Geometrie des kreiszylindrischen Verankerungsteils, dessen Durchmesser konstant ist und lediglich im obersten, dem Gelenkteil zugewandten Abschnitt in einen kurzen Konus übergeht. Ein weiterer Nachteil besteht darin, dass der distale vom Gelenkteil abgewandte Abschnitt der Verankerungsteils mit einem starken Gewinde versehen ist und keinerlei Perforationen im starkwandigen Hohlzylinder vorgesehen sind.

Hier will die Erfindung Abhilfe schaffen. Der Erfindung liegt die Aufgabe zugrunde, eine Schaftkomponente zu schaffen, welche einfach implantierbar ist, eine optimale Adaptation an die anatomischen Verhältnisse bewirkt und auch noch intraoperativ oder gegebenenfalls sogar postoperativ eine Änderung der Halsgeometrie (Antetorsion, Halsschaftwinkel, Halslänge, Trochanterersatz) gestattet, ohne den bereits implantierten Verankerungsteil des Schaftes wieder entfernen oder adaptieren zu müssen.

Die Erfindung löst die gestellte Aufgabe mit einer Schaftkomponente, welche die Merkmale des Anspruchs 1 aufweist.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Schaftkomponente eine optimale Adaptation an die anatomischen Verhältnisse erreichbar ist und die Lagerhaltung, dank des angewandten Baukastenprinzips auf ein funktionelles Minimum reduziert werden kann.

Die Erfindung und Weiterbildungen der Erfindung werden im folgenden anhand eines Ausführungsbeispiels einer Schaftkomponente für das Hüftgelenk näher erläutert. Die Erfindung kann aber auch für Schaftkomponenten anderer Endoprothesen, z.B. Schulter- oder Fingerprothesen, Anwendung finden.

Es zeigen:
Fig. 1 eine perspektivische, explosionsartige Darstellung der erfindungsgemässen Schaftkomponente; und
Fig. 2 einen Längsschnitt durch die im Femur implantierte, zusammengesetzte Schaftkomponente nach Fig. 1.

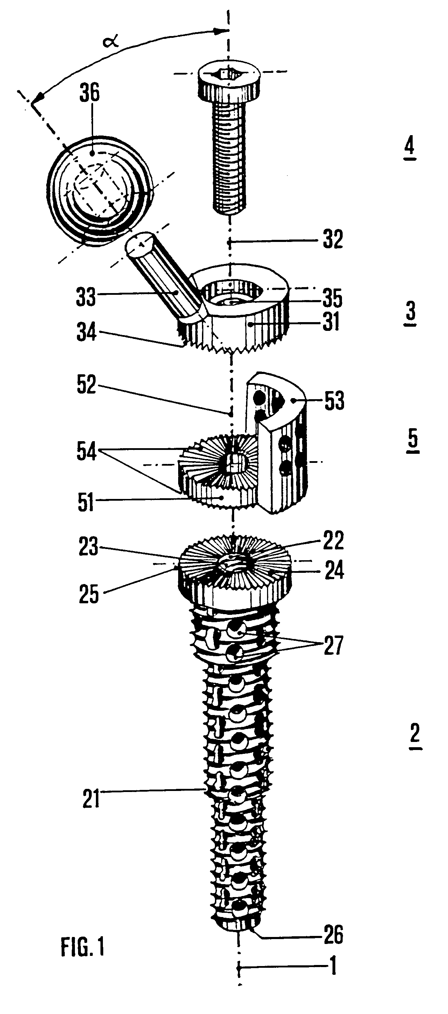

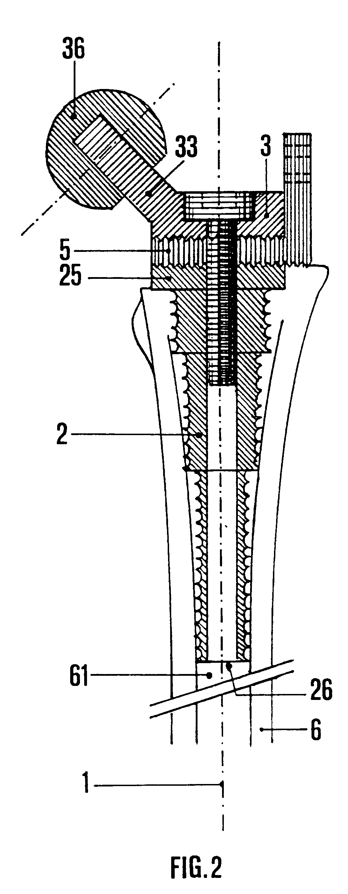

Die in den Fig. 1 und 2 dargestellte Schaftkomponente für eine Hüftgelenkprothese besteht im wesentlichen aus einem hohlzylinderförmigen, eine Längsachse 1 aufweisenden, rotationssymmetrischen Verankerungsteil 2 und einem separaten am Verankerungsteil 2 lösbar befestigbaren Gelenkteil 3.

Das Verankerungsteil 2 weist an seinem oberen dem Gelenkteil 3 zugewandten Ende 25 einen senkrecht zur Längsachse 1 angeordneten Zahnkranz 24, sowie eine zentrale Bohrung 22 mit einem, einen konstanten Durchmesser aufweisenden Innengewinde 23 auf.
Gegen das andere, in die Markraumhöhle 61 des Femur 6 einzuführende Ende 26 hin verjüngt sich das Verankerungsteil 2 in Form teleskopartiger, koaxialer Hohlzylinderabschnitte, was zu einer optimalen Anpassung an die Anatomie der Markraumhöhle 61 führt. Das Verankerungsteil 2 hat im weiteren eine strukturierte Oberfläche in Form eines Gewindes 21 sowie von Perforationen 27, welche das Einwachsen von Knochenmaterial fördern. Statt der Perforationen und des Gewindes oder zusätzlich zu diesen Strukturen kann die Oberfläche auch Längsrillen aufweisen, welche zur Längsachse (1) parallel verlaufen, oder mit einer Beschichtung oder Oberflächenätzung versehen sein. Zweck dieser Strukturierungen ist ein verbessertes Anwachsen des Knochens.

Das Gewinde 21 ist für den Knochen selbstschneidend ausgebildet, wodurch sich die Verwendung eines separaten Gewindeschneiders - wie er für konventionelle Prothesen erforderlich ist - erübrigt.

Das Gelenkteil 3 besteht im wesentlichen aus einem rotationssymmetrischen, ringförmigen Grundkörper 31 mit einer Rotationsachse 32 und einem, gegenüber dem Grundkörper 31 exzentrisch angeordneten, unter einem Winkel α von 70° zur Rotationsachse 32 verlaufenden, vorzugsweise konisch ausgebildeten Halsfortsatz 33 zur Aufnahme einer Gelenkkugel 36. Die Gelenkkugel kann auch fest mit dem Halsfortsatz 33 verbunden sein.
Das Gelenkteil 3 weist an seinem unteren, dem Verankerungsteil 2 zugewandten Ende einen senkrecht zu seiner Längsachse 32 angeordneten Zahnkranz 34 auf, dessen Zähne den gleichen Winkelabstand aufweisen wie beim Zahnkranz 24 des Verankerungsteiles 2. Dadurch ist es möglich das Gelenkteil 3 mit seinem Grundkörper 31 derart auf das Verankerungsteil 2 aufzusetzen, dass sich seine Rotationsachse 32 mit derjenigen des Verankerungsteils 2 deckt und es mittels der, durch die zentrale Bohrung 35 des Gelenkteils 3 einführbaren Schraube 4 in jeder beliebigen Winkelposition relativ zur Längsachse 1 des Verankerungsteils 2 lösbar mit letzterem zu fixieren, so dass die radiale Richtung des Halsfortsatzes 33 zur Einstellung der Antetorsion relativ zum Verankerungsteil 2 wählbar veränderbar ist.
Die Schraube 4 muss zu diesem Zweck ein zum Innengewinde 23 des Verankerungsteils 2 korrespondierendes Aussengewinde aufweisen.
Statt dieser in Fig. 1 dargestellten Fixationsart mit einer Schraube 4, können auch andere Konstruktionen verwendet werden, z.B. in Form von Steck- oder Schnappverbindungen oder von Bajonettverschlüssen.

Bei einer besonderen Ausführungsform der Erfindung, wie sie in Fig. 1 und 2 dargestellt ist, kann ein zusätzliches, als Trochanterersatz dienendes, zwischen Gelenkteil 3 und Verankerungsteil 2 angeordnetes Teil 5 vorgesehen sein. Es umfasst im wesentlichen einen rotationssymmetrischen Grundkörper 51 mit einer Rotationsachse 52 und einem gegenüber dem Grundkörper 51 exzentrisch angeordneten Zylindermantelsektor 53.
Der Grundkörper 51 ist an seinem oberen, dem Gelenkteil 3 zugewandten Ende und an seinem unteren, dem Verankerungsteil 2 zugewandten Ende mit je einem Zahnkranz 54 versehen, der mit den Zahnkränzen 24 und 34 korrespondiert. Dadurch wird es möglich, auch dieses Teil 5 in jeder beliebigen radialen Position sowohl zum Verankerungsteil 2, als auch zum Gelenkteil 3 lösbar zu fixieren.

Dieses zusätzliche Teil 5 kann auch weggelassen werden, wenn kein Trochanterersatz notwendig ist.

Im weiteren ist es möglich den Teil 5 auch derart auszubilden, dass er als Verlängerungsstück zwischen den Teilen 2 und 3 dient. Eine solche Ausführung ist insbesondere bei überlangen Femurschäften angebracht, welche bis ins Kniegelenk reichen. In einem solchen Fall ist es sogar möglich in dem vom Gelenkteil 3 abgewandten Ende 26 des Verankerungsteils 2 eine Bohrung mit einem Innengewinde vorzusehen, in welcher dann ein Fixationsteil oder ein Teil der Kniegelenkprothese befestigbar ist.

## Patentansprüche

1. Schaftkomponente für eine Endogelenkprothese zur Implantation in einen Röhrenknochen, mit einem hohlzylinderförmigen, eine Längsachse (1) aufweisenden Verankerungsteil (2) und einem Gelenkteil (3), wobei
A) das Gelenkteil (3) als separates am Verankerungsteil (2) lösbar befestigbares Teil ausgebildet ist;
B) das Gelenkteil (3) einen im wesentlichen rotationssymmetrischen Grundkörper (31), eine Rotationsachse (32) und einen gegenüber dem Grundkörper (31) exzentrisch angeordneten, unter einem Winkel α kleiner als 90° zur Rotationsachse (32) verlaufenden Halsfortsatz (33) zur Aufnahme einer Gelenkkugel (36) aufweist;
C) das Gelenkteil (3) mit seinem Grundkörper (31) derart auf das Verankerungsteil (2) aufsetzbar und fixierbar ist, dass sich seine Rotationsachse (32) mit der Längsachse (1) deckt;
D) das Gelenkteil (3) in jeder beliebigen Winkelposition relativ zur Längsachse (1) des Verankerungsteils (2) auf diesem lösbar fixierbar ist, so dass die radiale Richtung des Halsfortsatzes (33) zur Einstellung der Antetorsion relativ zum Verankerungsteil (2) wählbar veränderbar ist; und
E) das Verankerungsteil (2) im wesentlichen rotationssymmetrisch ausgebildet ist,
**dadurch gekennzeichnet, dass**
F) der Verankerungsteil (2) sich in Richtung zum Gelenkteil (3) hin in Form koaxialer Hohlzylinderabschnitte, erweitert; und
G) das Verankerungsteil (2) mit Perforationen (27) versehen ist.

2. Schaftkomponente nach Anspruch 1, dadurch gekennzeichnet, dass das Gelenkteil (3) mittels eines Befestigungsmittels am Verankerungsteil (2) lösbar befestigbar ist.

3. Schaftkomponente nach Anspruch 2, dadurch gekennzeichnet, dass das Befestigungsmittel eine Schraube (4) ist.

4. Schaftkomponente nach einem der Ansprüche 1 bis 3, dadurch gekennzeichnet, dass das Verankerungsteil (2) eine strukturierte Oberfläche aufweist, vorzugsweise in Form eines Gewindes (21), von zur Längsachse (1) parallelen Längsrillen, einer Beschichtung oder Ätzung.

5. Schaftkomponente nach einem der Ansprüche 1 bis 4, dadurch gekennzeichnet, dass das Verankerungsteil (2) für den Knochen selbstschneidend ausgebildet ist.

6. Schaftkomponente nach einem der Ansprüche 1 bis 5, dadurch gekennzeichnet, dass das Verankerungsteil (2) eine zentrale Bohrung (22) aufweist, vorzugsweise mit einem, einen konstanten Durchmesser aufweisenden Innengewinde (23) zur Aufnahme einer Schraube (4).

7. Schaftkomponente nach einem der Ansprüche 1 bis 6, dadurch gekennzeichnet, dass das der Grundkörper (31) eine zentrale Bohrung (35) aufweist, vorzugsweise mit einer Abstufung zur Aufnahme des Kopfes einer Schraube (4).

8. Schaftkomponente nach einem der Ansprüche 1 bis 7, dadurch gekennzeichnet, dass das Verankerungsteil (2) an seinem oberen dem Gelenkteil (3) zugewandten Ende (25) mit einer senkrecht zur Längsachse (1) angeordneten strukturierten Fläche, vorzugsweise in Form eines Zahnkranzes (24) und der Gelenkteil (3) an seinem unteren, dem Verankerungsteil (2) zugewandten Ende mit einer senkrecht zur Längsachse (32) angeordneten strukturierten Fläche, vorzugsweise in Form eines Zahnkranzes (34) versehen ist.

9. Schaftkomponente nach Anspruch 8, dadurch gekennzeichnet, dass sich die Strukturierungen der beiden Flächen ergänzen und vorzugsweise die Zähne der beiden Zahnkränze (24,34) den gleichen Winkelabstand aufweisen.

10. Schaftkomponente nach einem der Ansprüche 1 bis 7, dadurch gekennzeichnet, dass das Verankerungsteil (2) an seinem oberen dem Gelenkteil (3) zugewandten Ende (25) mit einer senkrecht zur Längsachse (1) angeordneten glatten Fläche und der Gelenkteil (3) an seinem unteren, dem Verankerungsteil (2) zugewandten Ende mit einer senkrecht zur Längsachse (32) angeordneten strukturierten Fläche (34) versehen ist.

11. Schaftkomponente nach einem der Ansprüche 1 bis 7, dadurch gekennzeichnet, dass das Verankerungsteil (2) an seinem oberen dem Gelenkteil (3) zugewandten Ende (25) mit einer senkrecht zur Längsachse (1) angeordneten strukturierten Fläche und der Gelenkteil (3) an seinem unteren, dem Verankerungsteil (2) zugewandten Ende mit einer senkrecht zur Längsachse (32) angeordneten glatten Fläche (34) versehen ist.

12. Schaftkomponente nach einem der Ansprüche 1 bis 11, gekennzeichnet durch ein zusätzliches, als Knochenersatz, dienendes, zwischen Gelenkteil (3) und Verankerungsteil (2) angeordnetes Teil (5), das im wesentlichen einen rotationssymmetrischen, ringförmigen Grundkörper (51) mit einer Rotationsachse (52) umfasst, wobei der Grundkörper (51) an seinem oberen, dem Gelenkteil (3) zugewandten Ende und an seinem unteren, dem Verankerungsteil (2) zugewandten Ende vorzugsweise je mit einer Strukturierung versehen ist, die mit den strukturierten Flächen (24,34) korrespondiert und gegen diese lösbar fixierbar ist.

13. Schaftkomponente nach Anspruch 12, dadurch gekennzeichnet, dass das Teil (5) als Trochanterersatz ausgebildet ist und einen gegenüber dem Grundkörper (51) exzentrisch angeordneten Zylindermantelsektor (53) umfasst, wobei der Grundkörper (51) an seinem oberen, dem Gelenkteil (3) zugewandten Ende und an seinem unteren, dem Verankerungsteil (2) zugewandten Ende vorzugsweise je mit einem Zahnkranz (54) versehen ist, der mit den Zahnkränzen (24,34) korrespondiert.

14. Schaftkomponente nach einem der Ansprüche 1 bis 13, dadurch gekennzeichnet, dass der Winkel α zwischen 60° - 80°, vorzugsweise zwischen 65° - 75°, liegt.

15. Schaftkomponente nach einem der Ansprüche 1 bis 14, dadurch gekennzeichnet, dass das vom Gelenkteil (3) abgewandte Ende (26) des Verankerungsteils (2) eine Bohrung vorzugsweise mit einem Innengewinde aufweist, zur Befestigung eines zusätzlichen distalen Gelenkprothesenteils oder Fixationsteils.

## Claims

1. A shank unit for an endo joint-prosthesis to be implanted into a tubular bone having a hollow-cylindrical anchoring sub-assembly (2) with a longitudinal axis (1) and an articulating sub-assembly (3), whereby
A) the articulating sub-assembly (3) is a separate sub-assembly detachably affixed to the anchoring sub-assembly (2);
B) the articulating sub-assembly (3) comprises an essentially rotationally symmetric base element (31), an axis of rotation (32) and a neck extension (33) mounted eccentrically relative to the base element (31) and subtending an angle α less than 90° with the axis of rotation (32) and serving to receive a joint-ball (36);
C) the articulating sub-assembly (3) can be so mounted by means of its base element (31) on the anchoring sub-assembly (2) and be so fixed in place that its axis of rotation (32) coincides with the longitudinal axis (1);
D) the articulating sub-assembly (3) is detachably fixable to the anchoring sub-assembly (2) in any angular position relative to the longitudinal axis (1) onto said anchoring sub-assembly (2), so that the radial direction of the neck extension (33) adjusting the anterior torsion relative to the anchoring sub-assembly (2) is selectively adjustable; and
E) the anchoring sub-assembly (2) is substantially rotationally symmetric,
**characterized in that**
F) the anchoring sub-assembly (2) widens in the direction toward the articulating sub-assembly (3) in the form of coaxial, hollow cylindrical segments; and
G) the anchoring sub-assembly (2) is provided with perforations (27).

2. A shank unit according to claim 1, characterized in that the articulating sub-assembly (3) is detachably fixable to the anchoring sub-assembly (2) by means of a fixing means.

3. A shank unit according to claim 2, characterized in that the fixing means is a screw (4).

4. A shank unit according to one of the claims 1 to 3, characterized in that anchoring sub-assembly (2) evinces a structured surface preferably in the form of a thread (21) , or of longitudinal channels parallel to the longitudinal axis (1), of a coating or of an etching.

5. A shank unit according to one of the claims 1 to 4, characterized in that anchoring sub-assembly (2) is self tapping into the bone.

6. A shank unit according to one of the claims 1 to 5, characterized in that anchoring sub-assembly (2) comprises a central borehole (22) preferably evincing an inner thread (23) of constant diameter to receive a screw (4).

7. A shank unit according to one of the claims 1 to 6, characterized in that the base element (31) comprises a central borehole (35) preferably with an offset to receive the head of a screw (4).

8. A shank unit according to one of the claims 1 to 7, characterized in that the anchoring sub-assembly (2) comprises at its upper end (25) facing the articulating sub-assembly (3) a structured surface perpendicular to the longitudinal axis (1) and preferably in the form of a serrated disk surface (24) and in that the articulating sub-assembly (3) comprises at its lower end facing the anchoring sub-assembly (2) a structured surface, perpendicular to the longitudinal axis (32) and preferably in the form of a serrated disk surface (34).

9. A shank unit according to claim 8, characterized in that the structures of the two surfaces are complementary and in that preferably the serrations of the two serrated disk surfaces (24,34) evince the same angular spacings.

10. A shank unit according to one of the claims 1 to 7, characterized in that the anchoring sub-assembly (2) comprises at its upper end (25) facing the articulating sub-assembly (3) a smooth surface perpendicular to the longitudinal axis (1) and in that the articulating sub-assembly (3) comprises at its lower end facing the anchoring sub-assembly (2) a structured surface (34) perpendicular to the longitudinal axis (32).

11. A shank unit according to one of the claims 1 to 7, characterized in that the anchoring sub-assembly (2) comprises at its upper end (25) facing the articulating sub-assembly (3) a structured surface perpendicular to the longitudinal axis (1) and in that the articulating sub-assembly (3) comprises at its lower end facing the anchoring sub-assembly (2) a smooth surface (34) perpendicular to the longitudinal axis (32).

12. A shank unit according to one of the claims 1 to 11, characterized in an additional part (5), serving as a bone replacement, between the articulating sub-assembly (3) and the anchoring sub-assembly (2) and essentially consisting of a rotationally symmetrical annular base element (51) with an axis of rotation (52), said base element (51) preferably comprising at its upper and lower ends respectively facing the articulating sub-assembly (3) and the anchoring sub-assembly (2) a structure matching the structured surfaces (24,34) to which said structure is detachably fixable.

13. A shank unit according to claim 12, characterized in that the part (5) is designed as a trochanter replacement and comprises a cylindrical case segment (53) which is eccentric relative to the base element (51), said element (51) preferably comprising at each of its upper and lower ends respectively facing the articulating sub-assembly (3) and the anchoring sub-assembly (2) a serrated disk-surface (54) matching the disk surfaces (24,34).

14. A shank unit according to one of the claims 1 to 13, characterized in that the angle α ranges from 60° to 80°, preferably between 65° and 75°.

15. A shank unit according to one of the claims 1 to 14, characterized in that the end (26) of the anchoring sub-assembly (2) away from the articulating sub-assembly (3) comprises a borehole preferably with an inner thread to affix an additional distal joint-prosthesis part or a fastener.

## Revendications

1. Tige pour une endoprothèse articulaire destinée à être implantée dans un os tubulaire et ayant une partie d'ancrage (2) à cylindre creux avec une axe longitudinale (1) et une partie articulaire (3), où
A) la partie articulaire (3) est une partie distincte fixée de manière amovible à la partie d'ancrage (2);
B) la partie articulaire (3) comprend un élément de base (31) essentiellement symétrique rotatif, une axe de rotation (32) et une extension de col (33) monté de manière excentrique relatif à l'élément de base (31) et englobant un angle α de moins de 90° avec l'axe de rotation (32) et servant à recevoir une tête articulaire (36);
C) la partie articulaire (3) peut être montée et fixée au moyen de son élément de base (31) sur la partie d'ancrage (2) de manière que son axe de rotation (32) coïncide avec l'axe longitudinale;
D) la partie articulaire (3) peut être fixée de manière amovible à la partie d'ancrage (2) dans toute position angulaire désirée relative à l'axe longitudinale (1), de manière que la direction radiale de l'extension de col (33) peut être changée de manière sélective pour ajuster l'antétorsion relative à la partie d'ancrage (2); et
E) la partie d'ancrage (2) est essentiellement symétrique rotative,
**caractérisée en ce que**
F) la partie d'ancrage (2) grandit dans la direction de la partie articulaire (3) en forme de segments coaxials de cylindre creux; et
G) la partie d'ancrage (2) est pourvue de perforations (27).

2. Tige selon la revendication 1, caractérisée en ce que la partie articulaire (3) peut être fixée de manière amovible à la partie d'ancrage (2) avec des moyens de fixation.

3. Tige selon la revendication 2, caractérisée en ce que le moyen de fixation est une vis (4).

4. Tige selon une des revendications 1 à 3, caractérisée en ce que la partie d'ancrage (2) dispose d'une surface structurée préférablement en forme d'un filetage (21), ou de rainures longitudinales parallèles à l'axe longitudinale (1), d'une couche appliquée à sa surface ou d'un procédé de corrosion appliqué à sa surface.

5. Tige selon une des revendications 1 à 4, caractérisée en ce que la partie d'ancrage (2) est auto-taraudent dans l'os.

6. Tige selon une des revendications 1 à 5, caractérisée en ce que la partie d'ancrage (2) comprend une forure centrale (22) préférablement ayant un filetage intérieur (23) de diamètre constant pour recevoir une vis (4).

7. Tige selon une des revendications 1 à 6, caractérisée en ce que l'élément de base (31) comprend une forure centrale (35) préférablement ayant une gradation pour recevoir la tête d'une vis (4).

8. Tige selon une des revendications 1 à 7, caractérisée en ce que la partie d'ancrage (2) comprend à sa partie terminale supérieure (25) adjacent à la partie articulaire (3) une surface structurée perpendiculaire à l'axe longitudinale (1) et préférablement en forme de couronne dentée (24) et que la partie d'ancrage (2) comprend à sa partie terminale inférieure (25) adjacent à la partie d'ancrage (2) une surface structurée perpendiculaire à l'axe longitudinale (32) et préférablement en forme de couronne dentée (34).

9. Tige selon la revendication 8 caractérisée en ce que les structures des deux surfaces structurées sont complémentaires et en ce que préférablement les dents des couronnes dentées (24,34) ont la même distance angulaire.

10. Tige selon une des revendications 1 à 7, caractérisée en ce que la partie d'ancrage (2) comprend à sa partie terminale supérieure (25) adjacent à la partie articulaire (3) une surface lisse perpendiculaire à l'axe longitudinale (1) et préférablement en forme de couronne dentée (24) et que la partie d'ancrage (2) comprend à sa partie terminale inférieure adjacent à la partie d'ancrage (2) une surface structurée (34) perpendiculaire à l'axe longitudinale (32).

11. Tige selon une des revendications 1 à 7, caractérisée en ce que la partie d'ancrage (2) comprend à sa partie terminale supérieure (25) adjacent à la partie articulaire (3) une surface structurée perpendiculaire à l'axe longitudinale (1) et que la partie d'ancrage (2) comprend à sa partie terminale inférieure adjacent à la partie d'ancrage (2) une surface lisse (34) perpendiculaire à l'axe longitudinale (32).

12. Tige selon une des revendications 1 à 11, caractérisée par un élément additionnel (5), servant de remplacement ossuaire, entre la partie articulaire (3) et la partie d'ancrage (2) et consistant essentiellement d'un élément de base (51) symétrique rotatif avec une axe de rotation (52), ledit élément de base (51) comprenant préférablement à sa partie terminale supérieure adjacent à la partie articulaire (3) et a sa partie terminale inférieure adjacent à la partie d'ancrage (2) chacune une structure correspondent avec les surfaces structurées (24,34) et fixables de manière amovible contre lesdites surfaces structurées (24,34).

13. Tige selon la revendication 12, caractérisée en ce que la partie (5) est réalisée comme remplacement de trochanter et comprend un segment de surface de cylindre creux (53) qui est excentrique relatif à l'élément de base (51), ledit élément de base (51) comprenant préférablement à sa partie terminale supérieure adjacent à la partie articulaire (3) et a sa partie terminale inférieure adjacent à la partie d'ancrage (2) chacune une couronne dentée (54) correspondent avec les surfaces structurées (24,34).

14. Tige selon une des revendications 1 à 13, caractérisée en ce que l'angle α est compris entre 60° à 80°, préférablement entre 65° à 75°.

15. Tige selon une des revendications 1 à 14, caractérisée en ce que la partie terminale (26) de la partie d'ancrage (2) opposé à la partie articulaire (3) comprend une forure préférablement avec un filetage intérieur pour la fixation d'une partie distale additionnelle de prothèse de joint ou pour une partie de fixation.
